(19) Europäisches Patentamt
European Patent Office
Office européen des brevets

(11) Numéro de publication: **0 099 286**
**B1**

(12) **FASCICULE DE BREVET EUROPEEN**

(45) Date de publication du fascicule du brevet: **20.05.87**

(51) Int. Cl.⁴: **A 61 K 37/02,** C 07 K 5/08

(21) Numéro de dépôt: **83401376.5**

(22) Date de dépôt: **04.07.83**

(54) **Composé contenant un séquence tripeptidique du type thréonyl-lysyl-proline ou analogue; utilisable en tant qu'agent anti-inflammatoire, anti-allergique ou antalgésique.**

(30) Priorité: **02.07.82 FR 8212222**

(43) Date de publication de la demande:
**25.01.84 Bulletin 84/04**

(45) Mention de la délivrance du brevet:
**20.05.87 Bulletin 87/21**

(84) Etats contractants désignés:
**BE CH DE FR GB IT LI LU NL SE**

(58) Documents cités:

**L. CHEDID et al.: "IMMUNOSTIMULATION", 1980, pages 147-156, Springer-Verlag, Berlin, DE**

(73) Titulaire: **INSTITUT PASTEUR DE LILLE**
**Rue du Professeur Calmette BP 245**
**F-59019 Lille Cedex (FR)**
(73) Titulaire: **INSTITUT PASTEUR (FONDATION)**
**28, rue du Docteur Roux**
**F-75715 Paris Cedex 15 (FR)**

(72) Inventeur: **Capron, André**
**58, rue du Capitaine Jasmin**
**F-59133 Phalempin (FR)**
Inventeur: **Joseph, Michel**
**24, rue Desrousseaux**
**F-59800 Lille (FR)**
Inventeur: **Dautrevaut, Michel**
**30, rue Jean Jaurès**
**F-59170 Croix (FR)**
Inventeur: **Auriault, Claude**
**1, Place de Verdun**
**F-59310 Mouchin (Orchies) (FR)**
Inventeur: **Dessaint, Jean-Paul**
**Place de Verdun**
**F-59310 Mouchin (Orchies) (FR)**
Inventeur: **Fougereau, Michel**
**35, Boulevard Matheron**
**F-13288 Marseille (9) (FR)**

**EP 0 099 286 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

(74) Mandataire: **Gutmann, Ernest et al**
**S.C. Ernest Gutmann - Yves Plasseraud 67,**
**boulevard Haussmann**
**F-75008 Paris (FR)**

**Description**

L'invention concerne un composé tripeptide caractérisé par la séquence de résidus aminoacyles lévogyres de formule suivante:

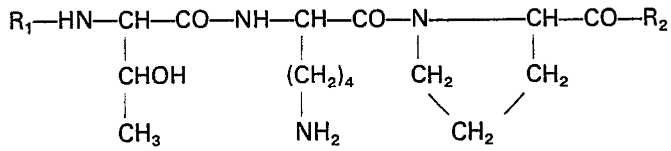

dans laquelle $R_1$ et un atome d'hydrogéne ou un groupe acétyle, glycolyle ou propionyle et $R_2$ est un groupe $—OH$, $—NH_2$, $—OCH_3$ ou $—OC_2H_5$.

L'invention concerne plus particulièrement le tripeptide L-thréonyl-L-lysyl-L-proline.

L'invention concerne encore les sels pharmaceutiquement acceptables de ces composés, qu'il s'agisse d'un sel d'addition d'acide ou d'un sel métallique, plus particulièrement alcalin. A titre d'exemple, on mentionnera à titre de sel alcalin, le sel de sodium du tripeptide sus-indiqué et, à titre d'exemple de sel d'addition d'acide, l'acétate du même tripeptide.

L'invention concerne également les dérivés résultant de la substitution des fonctions carboxyle-terminale et amine-terminale par d'autres groupes. A ce titre, l'invention concerne l'amide de la thréonyl-lysylproline de formule:

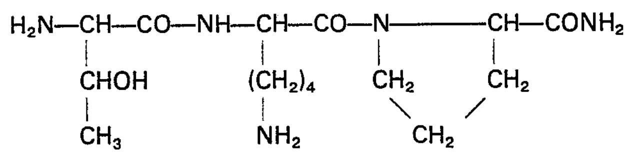

La thréonyl-L-lysyl-L-proline est un produit connu en tant que tel. Sa préparation a déjà été décrite, par exemple par Nishioka, Satok, Constantopoulos et Najjar dans Biochem. Biophys. Acta 1973, 310 (1), pp. 230—237, ou par Stabinsky, Gottlieb et Fridkin dans Mol. Cell. Biochem. vol. 30, No. 3, pp. 165—170. Néanmoins, aucune propriété ni pharmacologique ni thérapeutique n'a été révélée pour ce tripeptide. D'une manière classique, ce composé peut être obtenu suivant la technique de la phase solide de Merrifield, technique basée sur une synthèse progressive par additions successives des trois acides aminés.

Ce tripeptide a également été utilisé comme produit de comparaison dans les expériences décrites par E. Tsehoval et al, dans l'article intitulé "Immunostimulation by an IG derived tetrapeptide, Tuftsin" publié dans l'ouvrage intitulé "Immunostimulation" édité par D. Chedid et al, 1980, Springer Verlag, Berlin, Heidelberg, New York. Il y est rapporté que l'on observe chez le tripeptide Thr-Lys-Pro l'abolition de la propriété que possédait le tétrapeptide Thr-Lys-Pro-Arg, appelé tuftsine, d'activer les macrophages, lorque ceux-ci sont sollicités immunitairement et simultanément par un antigène et par le tétrapeptide.

L'invention concerne également ces composés pour leur utilisation en tant qu'agent anti-inflammatoires, anti-allergiques et/ou antalgiques. Ils se sont révélés capables d'inhiber l'activité phagocytaire des macrophages et neutrophiles et, à ce titre, peuvent être utilisés en thérapeutique. Ils inhibent les réactions d'hypersensibilité immédiate (allergies) induites chez l'hôte allergique exposé à des allergènes auxquels il est sensible. Il inhibe l'action de stimulation qu'exercent les allergènes chez les sujets allergiques, à l'égard de la production d'anticorps IgE, ainsi que des mécanismes impliqués dans les phénomènes allergiques, de dégranulation des mastocytes et basophiles. De même, l'invention se rapporte à des compositions pharmaceutiques ou vétérinaires contenant ces composés, notamment la L-thréonyl-L-lysyl-L-proline ou ses sels pharmaceutiquement acceptables, en association avec un véhicule pharmaceutique ou un excipient approprié.

Il est connu que les immunoglobulines de type G peuvent être adsorbées à la surface des formes larvaires de *Schistosoma mansoni*. De même, on a montré que ces immunoglobulines sont ensuite clivées par des enzymes protéolytiques d'origine parasitaire secrétées par la larve elle-même. Ce clivage a pour effet de libérer des peptides (solubles dans l'acide trichloroacétique à 6%) dans le milieu environnant le parasite, en maintenant en place la partie Fc des immunoglobulines (C. Auriault et Coll., Parasite Immunology 1981, *3*, 33—44).

L-intérêt particulier de ce phénomène est apparu lorsqu'il a été possible de mettre en évidence l'action fortement inhibitrice de ces peptides ainsi libérés vis-à-vis de l'activité des macrophages, c'est-à-dire des cellules effectives principales impliquées dans la réponse immunitaire dirigée contre le schistosome. La modulation de cette cellule par le parasite lui-même présente un intérêt particulier. Des études complémentaires ultérieures ont montré, en outre, que de façon générale l'activité phagocytaire des macrophages péritonéaux de rat ou alvéolaires humains est également déprimée de manière très significative en présence des produits résultant de l'hydrolyse d'IgG (hydrolysats d'IgG) par des protéase obtenues à partir de ces parasites, sans toutefois altérer la viabilité des cellules.

En effet, les mécanismes intervenant au cours de la phagocytose et plus particulièremente de la cytotoxicité anti-schistosomules des macrophages sont inhibés dans des proportions variant de 50 à 70%. De même, les tests basés sur la libération d'anion superoxyde $O_2^-$ responsable de la bactéricidie par les macrophages, l'incorporation de glucosamine, la libération de β-glucuronidase ainsi que la phagocytose de particules de latex radiomarquées montrent une inhibition dans les mêmes proportions (C. Auriault et Coll., Immunology Letters, 2, (1980), 135—139). Ces résultats indiquent que c'est essentiellement le mécanisme d'activation du macrophage qui est partiellement bloqué.

L'hydrolysat des IgG par les protéases parasitaires maintient les cellules à leur niveau de base d'activité quelle que soit la stimulation, par exemple induite par les réactions IgE-anti-IgE, IgG-anti-IgG, les peptides muramyl-dipeptide ou cyclomunine ou encore l'induction *in vivo* par la protéase peptone.

D'autre part, les macrophages alvéolaires de sujets allergiques stimulés par l'allergène correspondant sont également inhibés de manière significative (50 à 60%) s'ils ont été péalablement incubés avec des produits de l'hydrolyse des IgG par les protéases parasitaires.

Il a été trouvé, dans le cadre de l'invention, que la L-thréonyl-L-lysyl-L-proline correspondant à l'une des séquences peptidiques contenues à l'intérieur de la séquence Val-Gln-Val-His-Asn-Ala-Lys-Thr-Lys-Pro-Arg-Glu-Gln-Gln, appartenant à la région charnière des IgG I humaines, exerçait une activité inhibitrice semblable à l'égard de l'activité des macrophages. Cette activité est d'autant plus remarquable que la L-lysyl-L-prolyl-L-arginine n'est pas active et que le tétrapeptide Thr-Lys-Pro-Arg (tuftsine) présente au contraire une activité stimulante de l'activité immunogène des macrophages.

Selon des tests effectués par les méthodes décrites dans l'article cité plus haut de "Immunology Letters", la L'thréonyl-L-lysyl-L-proline s'est révélée atoxique pour les cellules et inhibitrice non seulement de la libération, mais également de la synthèse de la β-glucuronidase lysosomiale. En présence du peptide, le taux intracellulaire de cette enzyme décroît également. La L-thréonyl-L-lysyl-L-proline inhibe également la stimulation des macrophages de patients allergiques par l'allergène correspondant, lorsqu'ils ont été préincubés en présence du peptide. Cette dépression est très significative (50 à 70%) dans des essais *in vitro*. La thréonyl-L-lysyl-L-proline présente un pouvoir inhibiteur d'environ 60% aux concentrations de 200 nanogrammes à 20 picogrammes, ce pouvoir inhibiteur étant même de 50% à 2 picogrammes. Cette inhibition par le peptide se manifeste aussi dans des essais d'activation de macrophages par des réactions (IgE)-(Anti-IgE), que ces macrophages aient été incubés auparavant *in vitro* en présence du peptide, ou obtenus à partir d'animaux auxquels le peptide avait antérieurement été administré par voie intraveineuse (chez le rat, à des doses de 10 µg, trois heures avant le prélèvement des cellules). La libération d'anion superoxyde par les macrophages de rats est également inhibée par le tripeptide en question. De même, on observe dans des essais *in vitro*, une inhibition sensible de la dégranulation des mastocytes, notamment de rats, préalablement incubés en présence de la Thr-Lys-Pro, en présence de stimulants du type polymyxine ou phytohémagglutinine. Le peptide administré au rat induit également une inhibition de l'anaphylaxie cutanée passive provoquée chez l'animal par les administrations successives d'un antigène et de l'antisérum correspondant.

Les dérivés amidés et N-acétylés du tripeptide sus-défini possèdant essentiellement les mêmes propriétés, lesquells ont été confirmées au terme des mêmes tests. Cela s'applique plus particulièrement en ce qui concerne le dérivé amidé.

Les composés selon l'invention, tels quels ou leurs sels pharmaceutiquement acceptables, présentent des propriétés qui peuvent être mises en oeuvre sur le plan thérapeutique. Ils sont utilisables comme agents anti-inflammatoires, anti-allergiques ou antalgiques.

Les composés selon l'invention peuvent être synthétisés par des méthodes classiques en matière de synthèse peptidique. En raison de leur faible poids moléculaire, ils ne sont pas immunogènes. Ils sont biologiquement stables.

Les sels pharmaceutiquement acceptables, quant à eux, sont préparés de manière classique en traitant la L-thréonyl-L-lysyl-L-proline avec la quantité équivalente d'hydroxyde de métal alcalin en vue d'obtenir un sel de métal alcalin ou avec la quantité équivalente d'acide organique ou minéral approprié en vue d'obtenir un sel d'addition d'acide.

La L-thréonyl-L-lysyl-L-proline et ses sels pharmaceutiquement acceptables peuvent être administrés seuls mais plus généralement en association avec un véhicule pharmaceutique ou un excipient approprié.

De telles compositions thérapeutiques peuvent, être présentées sous toute forme convenant à l'administration en thérapie humaine ou vétérinaire. Pour ce qui concerne l'unité d'administration, celle-ci peut prendre la forme, par exemple d'un comprimé, d'une dragée, d'une capsule, d'une gélule, d'une poudre, d'une suspension ou d'un sirop pour l'administration orale, d'un suppositoire pour l'administration rectale, d'une solution stérile ou suspension pour l'administration parentérale ou d'une crème, d'un onguent, d'une lotion ou d'un gel pour l'administration topique.

De même, les compositions thérapeutiques en question pourront également se présenter sous forme d'une capsule ou d'un aérosol pour l'administration par voie respiratoire, d'une solution ou suspension pour l'administration nasale ou encore d'une solution ou suspension pour l'administration ophtalmique.

Suivant la voie d'administration choisie, les compositions thérapeutiques de l'invention seront préparées en associant la L-thréonyl-L-lysyl-L-proline ou un de ses sels pharamceutiquement acceptables, avec un véhicule ou un excipient approprié, ce dernier pouvant être constitué par exemple d'au moins un ingrédient sélectionné parmi des substances telles que l'eau distillée, l'alcool benzylique, le lactose, les

amidons, le talc, le stéarate de magnésium, la polyvinylpyrrolidone, l'acide alginique, la silice colloïdale ou les agents édulcorants.

La quantité de principe actif selon l'invention par unité d'administration variera selon que cette unité sera destinée à l'administration orale, rectale, parentérale, respiratoire, nasale ou autre. Dans le cas d'administration respiratoire, l'unité qui pourra être une capsule pour inhalation, comprendra par exemple de 10 à 30 mg par unité d'administration. Pour l'administration nasale, on pourra envisager par exemple des solution contenant de 1 à 3% de principe actif selon l'invention.

L'administration quotidienne de L-thréonyl-L-lysyl-L-proline ou de ses sels pharmaceutiquement acceptables dépendra de la voie d'administration choisie. Par exemple, pour l'administration par voie respiratoire, la quantité de ce principe actif pourra varier de 20 à 100 mg/jour à un être humain adulte, tandis que pour l'administration nasale ou ophtalmique, cette quantité pourra être de 10 à 30 mg/jour.

A titre d'exemple non limitatif, on décrit, ci-après, la préparation de la L-thréonyl-L-lysyl-L-proline et de ses sels pharmaceutiquement acceptables ainsi que de compositions thérapeutiques les contentant.

Exemple 1

L-thréonyl-L-lysyl-L-proline et ses sels

Sous agitation mécanique, on fait réagir pendant 24 h à 80°C, 13,8 mmol de $N^{im}$-t.BOC-proline (t-BOC=tert-butyloxycarbonyl) avec 20 g de résine chlorométhylée (résine de polystyrène-divinylbenzène chlorométhylée contenant 2,2 mmol de chlorure par gramme de résine) dans un mélange constitué de 12,4 mmol de triéthylamine et 30 ml d'éthanol. On lave la résine avec de l'acide acétique, puis avec de l'alcool absolu et on poursuit le lavage avec de l'eau contenant des quantités croissantes d'éthanol. On continue le lavage avec de l'éthanol, du méthanol et du chlorure de méthylène, puis on sèche la résine sous vide jusqu'à poids constant.

Dans un ballon équipé pour la réaction de Merrifield, on place 1,5 g de résine correspondant à 0,45 mmol et on élimine, à température ordinaire pendant une durée de 30 minutes, le groupement protecteur t-BOC avec 15 ml d'acide trifluoroacétique dans le chlorure de méthylène. On lave la résine avec du chlorure de méthylène puis avec trois fractions de 15 ml de chloroforme.

On ajoute à la résine 1 ml de triéthylamine et 9 ml de chloroforme et on agite pendant 10 minutes de façon à neutraliser le chlorhydrate. Après lavage au chloroforme et au chlorure de méthylène, on couple le $N^{im}$ de la proline pendant 2 heures et de façon continue avec 1,35 mmol de $N^{\alpha}$-t-BOC-$N^{\epsilon}$-carbobenzyloxy-lysine dans 15 ml de chlorure de méthylène et ce, au moyen de 1,35 mmol de dicyclohexylcarbodiimide. On lave la résine avec de l'ethanol, du chloroforme et du chlorure de méthylène en utilisant dans chaque cas trois fractions de 15 ml. On combine alors, de manière semblable au $N^{\alpha}$ de la lysine, après élimination du groupe protecteur et neutralisation, 1,35 mmol de $N^{\alpha}$-t-BOC-O-benzyl-thréonine et on répète les mêmes opérations ultérieures. On sépare le tripeptide de la résine en utilisant un mélange acide bromhydrique/acide trifluoroacétique à la température ambiante pendant 1,5 heure. On sèche sous vide, on lave à l'eau et on lyophilise. On reprend le produit obtenu dans 10 ml de méthanol contenant 10% d'acide acétique et on chromatographie ce mélange sur une résine connue sous la désignation Aminex®, en utilisant la pyridine comme éluant. On sèche le produit recueilli, on le lave deux fois avec de l'acide acétique 0,1 molaire, on le reprend dans l'acide acetique 0,1 molaire et on le lyophilise.

On obtient ainsi la L-thréonyl-L-lysyl-L-proline sous forme d'acétate, que l'on peut éventuellement faire réagir avec la quantité appropriée d'hydroxyde de sodium pour donner la L-thréonyl-L-lysyl-L-proline sous forme de sel sodique.

Le dérivé amidé de la L-thréonyl-L-lysyl-L-proline est obtenu par un procédé semblable, si ce n'est que le produit initial (la $N^{im}$-t-BOC-proline) est fixé au début de la synthèse sur une résine portant des groupes benzydrylamine et que, à l'occasion de la séparation ultérieure du dérivé tripeptidique formé de la résine, on utilise une solution d'acide fluorhydrique en lieu et place du mélange acide bromhydrique-acide trifluoroacétique. Cette façon de procéder permet d'obtenir directement le dérivé amidé.

Le dérivé de la L-thréonyl-L-lysyl-L-proline, acétylé au niveau de son groupe N-terminal, a été obtenu par N-acétylation du dérivé protégé de la L-thréonyl-L-lysyl-L-proline, avant la séparation de la résine et des groupes de protection.

Exemple 2

Suivant des techniques pharmaceutique connues, on a préparé les compositions suivantes:

a) Capsule pour administration respiratoire:

| | |
|---|---|
| L-thréonyl-L-lysyl-L-proline (sel sodique) | 20 mg |
| Lactose | 30 mg |
| | 50 mg |

b) Solution pour administration nasale

| | |
|---|---|
| L-thréonyl-L-lysyl-L-proline (sel sodique) | 2000 mg |
| Chlorure de benzalkonium | 10 mg |
| EDTA sodique | 10 mg |
| Eau distillée, pour compléter à | 100 ml |

Comme il va de soi et comme il résulte d'ailleurs déjà de ce que précède l'invention ne se limite nullement à ceux de ses modes d'application et de réalisation qui ont été plus spécialement envisagés; elle en embrasse au contraire toutes les variantes; parmi ces variantes figurent toutes les molécules résultant de la modification de la L-thréonyl-L-lysyl-L-proline et qui en conservent les propriétés essentielles, notamment en ce qui concerne les possibilités d'inhibition de l'activité phagocytaire des macrophages et de la dégranulation des mastocytes, dans des conditions semblables à celles qui se présentent à l'occasion de l'induction d'une réaction de type allergique.

A titre de variant susceptible d;être envisagée, on mentionnera les produits résultant de l'estérification du groupe carboxyle de l'acide aminé C-terminal ou encore la N-acylation de l'acide aminé N-terminal, par exemple pour former des méthyl- ou éthyl-esters, ou encore la N-acylation de l'acide aminé N-terminal pour former un groupe N-acyle distinct du groupe N-acétyle, par exemple un groupe glycolyle ou propionyle.

Tous les composés comportant de telles modifications et présentant de telles propriétés constituent par conséquent des équivalents des produits envisagés dans les revendications, et ces composés, surtout lorsqu'ils sont envisagés dans une utilisation aux fins thérapeutiques qui ont été indiquées, entrent de ce fait dans le périmètre de protection conféré par les revendications.

**Revendications**

1. Composé pour l'utilisation, comme agent anti-inflammatoire, anti-allergique ou antalgique, constitué par le composé de formule:

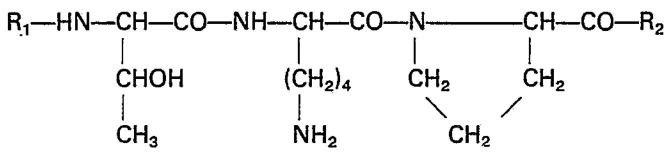

dans laquelle $R_1$ est un atome d'hydrogène ou un groupe acétyle, glycolyle ou propionyle et $R_2$ est un groupe $—OH$, $—NH_2$, $—OCH_3$ ou $—OC_2H_5$.

2. Compose pour l'utilisation comme agent pour l'inhibition de l'activité phagocytaire des macrophages ou pour l'inhibition de la dégranulation des mastocytes ou basophiles, constitué par le composé de formule:

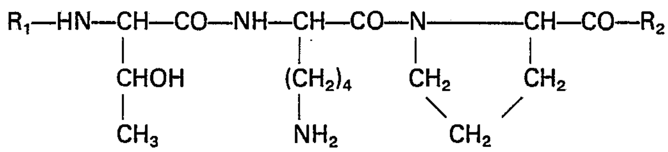

dans laquelle $R_1$ est un atome d'hydrogène ou une groupe acétyle, glycolyle ou propionyle et $R_2$ est un groupe $—OH$, $—NH_2$, $—OCH_3$ ou $—OC_2H_5$.

3. Composé selon la revendication 1 ou la revendication 2, caractérisé en ce qu'il est constitué par la L-thréonyl-L-lysyl-L-proline.

4. Composé selon la revendication 3, caractérisé en ce qu'il est à l'état de sel de metal alcalin ou de sel d'addition acide pharmaceutiquement acceptable.

5. Composé selon la revendication 1 ou la revendication 2, caractérisé en ce que $R_1$ est un atome d'hydrogène et $R_2$ est un groupe $NH_2$.

6. Compose selon la revendication 1 ou la revendication 2, caractérisé en ce que $R_1$ est un groupe acétyle et $R_2$ est un groupe hydroxyle.

7. Composition pharmaceutique contenant un composé selon l'une quelconque des revendications 1 à 6, en association avec un véhicule pharmaceutiquement acceptable.

8. Application d'un composé conforme à l'une quelconque des revendications 1 à 7 à la fabrication de médicaments destinés à l'utilisation en tant que médicament anti-inflammatoire, antalgique ou anti-allergique.

**Patentansprüche**

1. Verbindung zur Verwendung als entzündungshemmender, antiallergischer oder analgetischer Wirkstoff, bestehend aus einer Verbindung der Formel

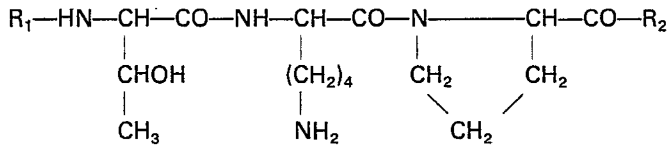

in der $R_1$ ein Wasserstoffatom oder eine Acetyl-, Glykolyl- oder Propionylgruppe bedeutet, und $R_2$ eine der Gruppen $—OH$, $—NH_2$, $—OCH_3$ oder $—OC_2H_5$ darstellt.

2. Verbindung zur Verwendung als Wirkstoff zur Inhibierung der Phagocytenwirkung von Makrophagen oder zur Inhibierung der Degranulation von Mastocyten und Basophilen, bestehend aus einer Verbindung der Formel

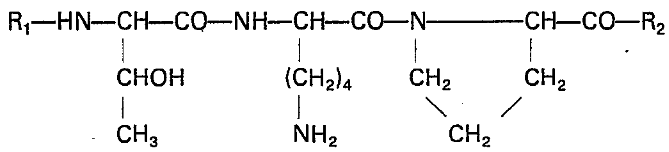

in der $R_1$ ein Wasserstoffatom oder eine Acetyl-, Glykolyl- oder Propionylgruppe bedeutet, und $R_2$ eine der Gruppen $—OH$, $—NH_2$, $—OCH_3$ oder $—OC_2H_5$ darstellt.

3. Verbindung nach Anspruch 1 oder Anspruch 2, dadurch gekennzeichnet, daß sie aus L-Threonyl-L-lysyl-L-prolin besteht.

4. Verbindung nach Anspruch 3, dadurch gekennzeichnet, daß sie in Form eines pharmazeutisch verträglichen Alkalimetallsalzes oder Säureadditionssalzes vorliegt.

5. Verbindung nach Anspruch 1 oder Anspruch 2, dadurch gekennzeichnet, daß $R_1$ ein Wasserstoffatom bedeutet und $R_2$ die Gruppe $NH_2$ darstellt.

6. Verbindung nach Anspruch 1 oder Anspruch 2, dadurch gekennzeichnet, daß $R_1$ eine Acetylgruppe bedeutet und $R_2$ eine Hydroxylgruppe darstellt.

7. Arzneimittel, enthaltend eine Verbindung nach einem der Ansprüche 1 bis 6 in Verbindung mit einem pharmazeutisch verträglichen Träger.

8. Anwendung einer Verbindung nach einem der Ansprüche 1 bis 7 zur Herstellung von Arzneimitteln, die zur Verwendung als entzündungshemmende, analgetische oder antiallergische Medikamente bestimmt sind.

**Claims**

1. Compound for use as an anti-inflammatory anti-allergic or antalgic agent constituted by the compound of formula:

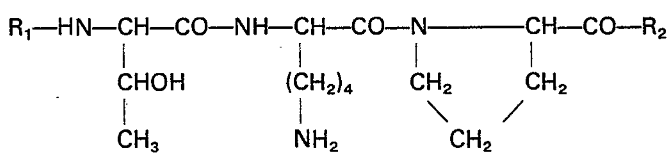

in which $R_1$ is an hydrogen atom or an acetyl, glycolyl or propionyl group and $R_2$ is a $—OH$, $—NH_2$, $—OCH_3$ or $—OX_2H_5$ group.

2. Compound for use as a reagent for inhibiting the phagocytic activity of the macrophages or for inhibiting the degranulation of the mastocytes or basophiles constituted by the compound of formula:

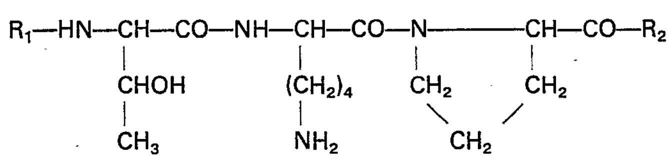

wherein $R_1$ is a hydrogen atom of an acetyl, glycolyl or propionyl group and $R_2$ is a group $—OH$, $—NH_2$, $—OCH_3$ or $—OC_2H_5$.

3. Compound according to claim 1 or claim 2 characterised in that it is constituted by the L-threonyl-L-lysyl-L-proline.

4. Compound according to claim 3, characterised in that it is under the form of an alkaline metal salt or of a pharmaceutically acceptable addition acid salt.

5. Compound according to claim 1 or claim 2, characterised in that $R_1$ is a hydrogen atom and $R_2$ is a group $NH_2$.

6. Compound according to claim 1 or claim 2, characterised in that $R_1$ is an acetyl group and $R_2$ is an hydroxyl group.

7. Pharmaceutical composition containing a compound according to any one of the claims 1 to 6, in association with a pharmaceutically acceptable vehicle.

8. Use of a compound according to anyone of the claims 1 to 7 for manufacturing medicaments intended to be used as anti-inflammatory, antalgic or anti-allergic medicaments.